# EUROPEAN PATENT SPECIFICATION

(11) **EP 4 167 952 B1**
(45) Date of publication and mention of the grant of the patent: **06.08.2025**
(21) Application number: 21724718.8
(22) Date of filing: 17.05.2021
(51) Int. Cl.: A61K 8/85, A61K 8/88, A61K 8/37, A61K 8/34, A61K 8/368, A61Q 90/00, A41G 3/00

(54) **BIODEGRADABLE ADHESIVE COMPOSITION**
BIOLOGISCH ABBAUBARE KLEBSTOFFZUSAMMENSETZUNG
COMPOSITION ADHÉSIVE BIODÉGRADABLE

(30) Priority: 22.06.2020 EP 20181327
(43) Date of publication of application: 26.04.2023
(73) Proprietor: Unilever IP Holdings B.V., 3013 AL Rotterdam (NL); Unilever Global IP Limited, Wirral, Merseyside CH62 4ZD (GB)
(72) Inventor: FLYNN, Joanna, New York 11735 (US); HAWKINS, Geoffrey, Robert, Victor, New York 11735 (US)
(74) Representative: Mathai, Neenu Grace
(86) International application number: PCT/EP2021/062934
(87) International publication number: WO 2021/259554

(56) References cited:
- EP-A1- 1 870 237
- DE-A1- 10 261 786
- DE-A1- 10 343 961
- GB-A- 2 451 883
- GB-A- 2 463 735

## Description

### Field of the Invention

Disclosed herein is an adhesive composition, for adhering an object such as a wig, extension or weave to human skin. The adhesive composition includes a biodegradable polymer, i.e., sodium polyitacone, a preservative, and water. The adhesive composition itself is biodegradable.

### Background of the Invention

Protective hair styles such as wigs, weaves, and/or extensions can be used to change hairstyles while leaving the user's natural hair untouched or undamaged. Various mechanisms can be utilized to attach the protective hair styles to human skin. Such mechanisms can include fasteners, combs, clips, adhesives, etc. In some applications, the adhesive needs to be cured after application, meaning that heat must be applied to adhesive after it has been attached to the user's scalp in order to activate and set. In other applications, an already heated adhesive is applied. A risk of burning the user's scalp or the applier's hands is always present when using an already heated adhesive. Another method includes the use of gels to attach the protective hair styles to human skin.

Such methods of application have several drawbacks. For example, fasteners, combs, and clips can come lose and detach the protective hair style from the scalp. Adhesives that need to be cured can leave lumps, which can be seen and felt and are often uncomfortable for the user. As previously mentioned, the use of hot adhesives brings the risk of burning the user or the person applying the hot adhesive. Such methods of application can also be very time consuming. Furthermore, as protective hair styles are not permanent, removal of the protective hair style can also pose problems. Fasteners, combs, and clips can remove a user's natural hair along with the protective hair style upon removal. Cured adhesives and the use of hot adhesives can require a solvent, such as a chemical solvent to release the bond with the user. Other times, water can be used to dissolve the bond.

As such, there is continually a need for a mechanism that can attach a protective hair style to a user that is not overly time consuming, that provides adhesion for a desired period of time, and that does not require harsh chemical solvents, or another liquid to remove the bond between the user the protective hair style.

UK Patent Application No. GB 2463735 discloses a range of fully biodegradable and compostable adhesives, which will find major use in attaching labels and tapes to the packaging of supermarket goods. The adhesives comprise a proprietary copolymer formed by polymerization of an alkyl polyglycoside maleic acid ester with a vinyl monomer mixed with an aliphatic hydrocarbon resin dispersion.

German Patent Application No. DE 103 43 961 A1 discloses a nontoxic pressure-sensitive based on natural raw materials and comprises 0.1 to 5 wt.% monosaccharide and 80-99.9 wt.% polysaccharide.

European Patent Application No. EP 1 870 237 A1 discloses a laminate sheet that is excellent in oxygen-barrier property and moisture resistance, biodegradable as a whole and therefore suitable as a base material for packaging materials, such as food containers. The laminate sheet is formed by laminating a water-containable and biodegradable polymer substrate sheet or a precursor thereof in a water-containing state with a layer of polyglycolic acid resin having a residual monomer content below 0.5 wt.% to form a laminate and subjecting the laminate to bonding and forming under heat and pressure.

UK Patent Application No. GB 2 451 882 A discloses a range of biodegradable and compostable adhesives useful in the packaging of supermarket goods. The adhesives comprise polyester or acrylic polymers, which have been modified by copolymerization with a range of aldo-pentoses and polyhydric alcohols to increase their biodegradability. Such additives include acrylic sugar-based macromers.

German Patent Application No. DE 102 61 786 A1 discloses a gelatin based adhesive that is sold at normal temperature, useful for the production of multi-layer tissues and paper towels and contains a water soluble polymer that surrounds the adhesive with fiber-drawing properties.

### Summary of the Invention

Disclosed in various aspects are adhesive compositions.

An adhesive composition for adhering an object such as a wig, extension or weave to human skin comprises about 2.5% to about 12% by weight of sodium polyitaconate, about 0.5 to about 1.5% by weight of a preservative, and at least 10% of water.

These and other features and characteristics are more particularly described below.

### Detailed Description of the Invention

Disclosed herein is an adhesive composition, more specifically, a biodegradable adhesive composition. The adhesive composition includes a biodegradable polymer, a preservative, and water. It was unexpectedly discovered that this combination of ingredients, comprising a biodegradable polymer, could provide adhesion between two objects for at least one week and even up to two weeks. This was unexpected since the biodegradable polymer is water soluble. Films formed from biodegradable polymers tend to plasticize and decrease the viscosity. Such plasticization was not observed with the present compositions. The biodegradable polymer can form a film and provide adhesion of a protective hair style such as a wig, weave, or extension to human skin and can also trap and contain any humidity therein.

The adhesive composition provides desired skin/hair feel to the consumer without film or barrier disruption. The adhesive properties are achieved without the use of latex, which is normally found in glues. The biodegradable composition can advantageously be removed without the use of toxic ingredients, such as solvents (e.g., formaldehyde), and contains no synthetic polymers. Optionally, skin comfort ingredients can be included in the composition to provide a more comfortable fit for the user. When the user is ready to change hairstyles or the adhesion has partially wom off, the user can simply wash their hair and scalp with a mild surfactant-based product, e.g., shampoo, to release the bond and remove the film. The film formed by the biodegradable polymer does not wear off and does not build-up on hair or skin, which could otherwise lead to increased entanglement, weighing down of the hair, or disruption to the skin stratum corneum barrier.

The adhesive composition disclosed herein can allow the user to shower and/or exercise 24 hours after application. The adhesive composition can also provide an invisible bond between the user and object to which it is adhered. The adhesive composition can dry clear with no flaking and healthy edges, meaning that edge hairs are not broken. Because a biodegradable polymer is used, the adhesive composition can be gentler to the user's skin without causing irritation. The adhesive composition disclosed herein offers many advantages compared to hot adhesives, gels, and curable adhesives.

The adhesive composition comprises a biodegradable polymer. The biodegradable polymer is present in an amount of about 2.5 to about 12% by weight of the composition including all ranges subsumed therein. For example, the biodegradable polymer can be present in an amount of about 4 to about 10% by weight, for example, the biodegradable polymer can be present in an amount of about 5 to about 7% by weight of the composition.

Biodegradable polymers are generally a special class of polymer that breaks down after its intended purpose by bacterial decomposition. Various natural by-products can be released including water, biomass, and inorganic salts. Biodegradable polymers can be found both naturally and synthetically, and largely comprise ester, amide, and ether functional groups. Their properties and breakdown mechanism are determined by their exact structure. These polymers are often synthesized by condensation reactions, ring opening polymerization, and metal catalysts.

The biodegradable polymer for use in the composition is sodium polyitaconate. It was unexpectedly found that the combination of a biodegradable polymer, such as sodium polyitaconate, with a preservative and water demonstrated a synergistic effect to provide an adhesive hold of a protective hair style to human skin.

The adhesive composition additionally comprises a preservative. Preservatives can desirably be incorporated into the adhesive composition to protect against the growth of potentially harmful microorganisms. The preservative is present in an amount of about 0.5 to 1.5% by weight of the composition including all ranges subsumed therein, for example, about 0.75 to about 1% by weight of the composition.

Cosmetic chemists are familiar with appropriate preservatives and routinely choose them to satisfy the preservative challenge test and to provide product stability. Traditional preservatives for use include hydantoin derivatives and propionate salts. Other preservatives for use are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin and benzyl alcohol and mixtures thereof. Other preservatives include sodium benzoate, sodium dehydroacetate, chlorophenesin and decylene glycol. Other preservatives include trisodium ethylenediamine disuccinate (EDDS), tetra sodium iminodisuccinate (IDS), sodium gluconate, phytic acid, sodium phytate, tricalcium citrate, trisodium dicarboxymethyl alaninate, caproyl/capryloyl anhydro methyl glucamide and water, caprylyl glycol, ethyl lauroyl arginate hydrochloric acid and glycerin, ethyl lauroyl arginate hydrochloric acid and capryl glycol and glycerin, gluconolactone, glyceryl caprylate, lactic acid, p-anisic acid, pentylene glycol, sodium citrate, sorbitan caprylate, butylated hydroxytoluene (BHT), dilauryl thiodipropionate, octadecyl di-t-butyl-4-hydroxyhydrocinnamate, pentaerythrityl tetra-di-t-butyll hydrohydrocinnamate, vitamin E, or a combination thereof. The preservatives should be selected having regard for the use of the composition and possible incompatibilities between the preservatives and other ingredients in the adhesive composition.

Some preservatives include, but are not limited to, benzoic acid, levulinic acid, ansic acid, iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin and benzyl alcohol, sodium benzoate, sodium dehydroacetate, chlorophenesin, decylene glycol, hydroxyacetophenone alone or in a mixture with other preservatives, sodium benzoate, or a combination thereof. Other preservatives that can be used include benzoin gum, sapindus mukorossi (soap nut) fruit extract, or a combination thereof. Preferably, the preservative can be benzoic acid, citric acid, salicylic acid, phenoxyethanol, dehydroacetic acid and salts thereof, chlorphenesin, diols, caprylyl glyclol, diol/glycol compounds, caprylhydroxamic acid, or a combination thereof.

The adhesive composition can further comprise a thickening agent. The thickening agent can be present in an amount of about 0.01% to about 5% by weight of the composition, for example, about 0.05% to about 4% by weight of the composition, for example, about 0.1% to about 3% by weight of the composition, for example, about 0.2% to about 3% by weight of the composition, and any and all ranges subsumed therein. Desirable thickening agents include an anionic polymer-based thickener, preferably, for example, a polyacrylate based polymer or copolymer, or a combination thereof. Polysaccharides can be used. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Desirable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose).

Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

As mentioned, synthetic polymers are effective thickening agents. This category includes crosslinked polyacrylates such as the Carbomers, polyacrylamides such as Sepigel^{®} 305 and taurate copolymers such as Simulgel^{®} EG and Aristoflex^{®} AVC, the copolymers being identified by respective INCI nomenclature as sodium acrylate/sodium acryloyldimethyl taurate and acryloyl dimethyltaurate/vinyl pyrrolidone copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

Sodium hydroxypropyl starch phosphate, aluminum starch octenylsuccinate, tapioca starch, maltodextrin, xanthan gum, agar gum, guar gum, carrageenan gum, alginate gum, hydroxypropyl cellulose, hydroxypropyl methylcellulose, ethylcellulose, sodium carboxy methylcellulose, cellulose, polyethylene glycol (e.g., polyethylene glycol diester stearic acid), or a combination thereof are further examples of thickening agents for use in the present adhesive compositions. Such thickening agents are commercially available from the Dow Chemical Company or the Hallstar Company.

Preferably, the thickening agent comprises cellulose, polysaccharide gum, or a combination thereof, preferably wherein the thickening agent comprises hydroxy cellulose, hydroxyethyl cellulose, hydroxyethyl methylcellulose, methyl cellulose, xanthan gum, guar gum, or a combination thereof.

The adhesive composition can further comprise a conditioning agent. The conditioning agent is present in an amount of about 0.01% to about 30%, by weight of the composition, for example, about 0.05% to about 25% by weight of the composition, for example, about 0.1% to about 20% by weight of the composition, for example, about 0.5% to about 15% by weight of the composition, for example, about 1% to about 10% by weight of the composition, and any and all ranges subsumed therein. The conditioning agent can provide the adhesive composition with a pleasing texture to human skin. Conditioning agents can include occlusives, e.g., petrolatum, dimethicone, and the like; humectants, e.g., glycerin, propylene glycol, sorbitol, saccharides such as oligosaccharides such as com syrup, and the like; emollients and oils, e.g., trigylycerides, natural oils, lanolin, synthetic esters, and the like; proteins; silicones, e.g., dimethicone, cyclomethicone, amodimethicone, and the like; cationic surfactants, e.g., cetrimonium chloride, stearalkonium chloride, and the like; and polymers, e.g., cationic polymers such as polyquarterniums. Preferred conditioning agents include glycerine, oligosaccharides such as com syrup, urea, or a combination thereof. The conditioning agent can also comprise butylene glycol, propanediol, or a combination thereof. The composition can optionally comprise shea butter in an amount of about 0 to about 0.1% by weight, for example, about 0.01% by weight.

The composition comprises water in an amount of at least 10% by weight of the composition. For example, water can be present in an amount of about 10% to about 85% by weight of the composition, for example, about 25% to about 75% by weight of the composition, for example, about 30% to about 65% by weight of the composition, for example, about 25% by weight of the composition, for example, about 35% by weight of the composition, for example, about 45% by weight of the composition, for example, about 55% by weight of the composition, for example, about 65% by weight of the composition, for example, about 75 by weight of the composition, for example, about 85% by weight of the composition, and any and all ranges subsumed therein.

The adhesive composition can be made in any form that will allow it to be adhered to another object, e.g., human skin. For example, the adhesive composition can be made into a lotion, butter, gel, liquid, spray (aerosol or non-aerosol), or a combination thereof.

The adhesive composition can have a viscosity as measured according to ASTM D445 at 25°C of 60,000 to 90,000 centipoise. A pH of the adhesive composition can be 5 to 7.5, for example, 5.5 to 7, for example, 5.5.

The adhesive composition can be used to adhere an object to human skin. For example, a method of adhering an object, such as a wig, extension, or weave to human skin can include applying the adhesive composition as disclosed herein to human skin and allowing the adhesive composition to dry for a period of time. Once dry, an adhesive bond is formed with the human skin. The drying can occur for a period of 30 seconds to 15 minutes, for example, 45 seconds to 10 minutes, for example, 1 minute to 5 minutes, for example, 1 minute. The adhesive bond form can last for 5 days, 7 days, 10 days, 14 days, or even longer.

The adhesive composition disclosed herein provides an alternative adhering method to users desiring a more natural material to hold the protective hair style in place, one that does not require harsh chemicals or solvents to remove. The adhesive composition disclosed herein is able to be removed by simply washing the user's hair or skin with water and shampoo (or another cleaning material) in order to release the bond. The adhesive composition dries quickly, does not require heat to set, and is non-irritating to most user's skin. An unexpected synergy was found between the biodegradable polymer, preservative, and water to provide sufficient adhesion for a desired period of time.

### Examples

### Example 1

In this example, in vitro testing for 24-48 hours was conducted using bald-headed mannequins and human hair wigs both at an ambient temperature of 25°C and at an elevated temperature of 45°C.

A dose response was conducted using sodium polyitaconate and water to discover desirable concentration levels. Compositions were made with the formulations listed in Table 1 and the human hair wig adhered to the bald-headed mannequin. The sodium polyitaconate was varied from 1 to 10% by weight of the overall adhesive composition, while the water amount was adjusted to reflect these changes as shown in Table 2. Adhesion was tested by inverting the mannequin head and applying a weight or physically pulling on the wig to see if the adhesion would release. At sodium polyitaconate concentrations of 5 to 10% by weight of the overall adhesive composition, the wig did not release from the mannequin at either temperature.

**Table 1**

| **Material Description** | **% Weight** |
|---|---|
| Com Syrup | 20.0 |
| Hydroxyethyl Cellulose | 2.0 |
| Glycerine | 5.0 |
| Borage Oil | 0.01 |
| Tea Tree Oil | 0.01 |
| Shea Butter | 0.01 |
| Preservative (VERSTATIL^{®} TBO) | 1.5 |
| Fragrance | 0.3 |

**Table 2**

| **Sample #** | **Amount water (% weight)** | **Amount sodium polyitaconate (% weight)** |
|---|---|---|
| Ref. 1 | 70.17 | 1 |
| Ref. 2 | 69.17 | 2 |
| 3 | 68.17 | 3 |
| 4 | 67.17 | 4 |
| 5 | 66.17 | 5 |
| 6 | 65.17 | 6 |
| 7 | 64.17 | 7 |
| 8 | 63.17 | 8 |
| 9 | 62.17 | 9 |
| 10 | 61.17 | 10 |

Thus, it was found that a desirable concentration of sodium polyitaconate was between 5 to 10% by weight of the overall composition.

### Example 2

In this example, in vitro testing for 24-48 hours was conducted using bald headed mannequins and human hair wigs both at an ambient temperature of 25°C and at an elevated temperature of 45°C

Compositions were made as shown in Table 3 where all amounts are measured in % by weight of the composition, and the human hair wig adhered to the bald-headed mannequin. Adhesion was tested by inverting the mannequin head and applying a weight or physically pulling on the wig to see if the adhesion would release.

In this example, evaluations for hold of the adhesive composition disclosed herein were conducted versus traditional styling polymers such as polyvinylpyrrolidone (PVP) (Samples 11 and 12), which is a water-soluble polymer used for styling in most gels and mousses, com starch, and other branched chained polysaccharides such as guar, xanthan, and straight-chain carrageenan gum. PVP is a hydroscopic material, so it picks up moisture, thereby reducing hold. Hold is a very important tribute in heat and humidity. In Sample 13, polysaccharide polymer was used, Sample 14 used synthetic Polyquatemium 4, and Sample 15 used a combination of Polyquatemium 4 and arabica gum (total w/w of 5% of both). Samples 16 and 17 contained guar gum, Samples 18 and 19 contained xanthan gum, and Samples 20 and 21 contained a straight-chained carrageenan gum. The amount was varied between 3.0 to 6.0% by weight in Samples 16 to 21.

**Table 3**

| **Material** | **Ref. Sample 11** | **Ref. Sample 12** | **Ref. Sample 13** | **Ref. Sample 14** | **Ref. Sample 15** |
|---|---|---|---|---|---|
| Com Syrup | 20.0 | 20.0 | 20.0 | 20.0 | 20.0 |
| Hydroxyethyl Cellulose | 2.0 | 2.0 | 2.0 | 2.0 | 2.0 |
| Glycerine | 5.0 | 5.0 | 5.0 | 5.0 | 5.0 |
| Borage Oil | 0.01 | 0.01 | 0.01 | 0.01 | 0.01 |
| Tea Tree Oil | 0.01 | 0.01 | 0.01 | 0.01 | 0.01 |
| Shea Butter | 0.01 | 0.01 | 0.01 | 0.01 | 0.01 |
| Preservative (VERSTATIL^{®} TBO) | 1.5 | 1.5 | 1.5 | 1.5 | 1.5 |
| Fragrance | 0.3 | 0.3 | 0.3 | 0.3 | 0.3 |
| Water | 68.17 | 65.17 | 65.17 | 68.17 | 66.17 |
| PVP | 3.0 | 6.0 | - | - | - |
| Polysaccharide Polymer | - | - | 6.0 | - | - |
| Synthetic Polyquaternium 4 | - | - | - | 3.0 | |
| Synthetic Polyquaternium 4 and Arabica gum | | | | | 5.0 |

**Table 4**

| **Material** | **Sample 16** | **Sample 17** | **Sample 18** | **Sample 19** | **Sample 20** | **Sample 21** |
|---|---|---|---|---|---|---|
| Com Syrup | 20.0 | 20.0 | 20.0 | 20.0 | 20.0 | 20.0 |
| Hydroxyethyl Cellulose | 2.0 | 2.0 | 2.0 | 2.0 | 2.0 | 2.0 |
| Glycerine | 5.0 | 5.0 | 5.0 | 5.0 | 5.0 | 5.0 |
| Borage Oil | 0.01 | 0.01 | 0.01 | 0.01 | 0.01 | 0.01 |
| Tea Tree Oil | 0.01 | 0.01 | 0.01 | 0.01 | 0.01 | 0.01 |
| Shea Butter | 0.01 | 0.01 | 0.01 | 0.01 | 0.01 | 0.01 |
| Preservative (VERSTATIL^{®} TBO) | 1.5 | 1.5 | 1.5 | 1.5 | 1.5 | 1.5 |
| Fragrance | 0.3 | 0.3 | 0.3 | 0.3 | 0.3 | 0.3 |
| Water | 62.17 | 59.17 | 62.17 | 59.17 | 62.17 | 59.17 |
| Sodium polyitaconate | 6.0 | 6.0 | 6.0 | 6.0 | 6.0 | 6.0 |
| Guar Gum | 3.0 | 6.0 | - | - | - | - |
| Xanthan Gum | - | - | 3.0 | 6.0 | - | - |
| Straight-Chained Carrageenan Gum | - | - | - | - | 3.0 | 6.0 |

In the tests, longer lasting hold during the in vitro testing of 24-48 hours was observed with the adhesive composition disclosed herein comprising sodium polyitaconate of Samples 16 to 21 as compared to Reference Samples 11 to 15 at either temperature meaning that the wig did not release from the mannequin at either temperature. PVP and the combination of Polyquatemium 4 and arabica gum demonstrated no hold at either temperature. Polyquatemium 4 showed no hold at 45°C. Polysaccharide polymer show a weaker hold compared to sodium polyitaconate at either temperature.

### Example 3

In this example, in vitro testing for 24-48 hours was conducted using bald headed mannequins and human hair wigs both at an ambient temperature of 25°C and at an elevated temperature of 45°C.

The adhesive composition as disclosed herein and shown in Table 5 (Sample 22) were tested versus a commercially available product for hold. The commercially available composition (Reference Sample 23) contained PVP water, sorbitol, PVP, vinyl caprolactam/vinyl pyrrolidone/dimethylaminoethyl methacrylate copolymer, petrolatum, vinyl pyrrolidone/ dimethylaminopropyl methacrylamide acrylates copolymer, cetearyl alcohol, steareth-21, stearyl alcohol, cetyl alcohol, com starch modified, parfum (fragrance), dicetyl phosphate, ceteth-10 phosphate, dmdm hydantoin, cera alba (beeswax, cire d'abeille), hydroxyethylcellulose, propylene glycol, diazolidinyl urea, myristyl alcohol, arachidyl alcohol, benzalkonium chloride, iodopropynyl butylcarbamate, and polyaminopropyl biguanide.

After the composition was made with the formulation shown in Table 5, the human hair wig was adhered to the bald-headed mannequin. Adhesion was tested by inverting the mannequin head and applying a weight or physically pulling on the wig to see if the adhesion would release.

**Table 5**

| **Material Description** | **% Weight** |
|---|---|
| Com Syrup | 20.0 |
| Hydroxyethyl Cellulose | 2.0 |
| Glycerine | 5.0 |
| Borage Oil | 0.01 |
| Tea Tree Oil | 0.01 |
| Shea Butter | 0.01 |
| Preservative (VERSTATIL^{®} TBO) | 1.5 |
| Fragrance | 0.3 |
| Water | 65.17 |
| Sodium Polyitaconate | 6.0 |

Superior hold was observed with the adhesive composition comprising sodium polyitaconate at both temperatures.

### Example 4

In this example, an experiment was conducted to ensure that there are no adverse consequences with the inclusion of oligosaccharide com syrup. Two adhesive compositions were made as shown in Table 6, one containing com syrup and no sodium polyitaconate (Reference Sample 24) and one containing both com syrup and sodium polyitaconate (Sample 25). In this example, in vitro testing for 24-48 hours was conducted using bald headed mannequins and human hair wigs both at an ambient temperature of 25°C and at an elevated temperature of 45°C.

**Table 6**

| **Material** | **Reference Sample 24** | **Sample 25** |
|---|---|---|
| Com Syrup | 20.0 | 20.0 |
| Hydroxyethyl Cellulose | 2.0 | 2.0 |
| Glycerine | 5.0 | 5.0 |
| Borage Oil | 0.01 | 0.01 |
| Tea Tree Oil | 0.01 | 0.01 |
| Shea Butter | 0.01 | 0.01 |
| Preservative (VERSTATIL^{®} TBO) | 1.5 | 1.5 |
| Fragrance | 0.3 | 0.3 |
| Water | 71.17 | 65.17 |
| Sodium polyitaconate | - | 6.0 |

The compositions were made and the human hair wig adhered to the bald-headed mannequin. Adhesion was tested by inverting the mannequin head and applying a weight or physically pulling on the wig to see if the adhesion would release.

No adhesion was observed in the composition containing com starch and no sodium polyitaconate at ambient or at an elevated temperature of 45°C.

### Example 5

In this example, an experiment was conducted to ensure no adverse consequences with the inclusion of cellulose in the adhesive composition. Two adhesive compositions were made as shown in Table 7, one containing cellulose and no sodium polyitaconate (Reference Sample 26) and one containing both cellulose and sodium polyitaconate (Sample 27). In this example, in vitro testing for 24-48 hours was conducted using bald headed mannequins and human hair wigs both at an ambient temperature of 25°C and at an elevated temperature of 45°C.

**Table 7**

| **Material** | **Reference Sample 26** | **Sample 27** |
|---|---|---|
| Com Syrup | 20.0 | 20.0 |
| Hydroxyethyl Cellulose | 2.0 | 2.0 |
| Glycerine | 5.0 | 5.0 |
| Borage Oil | 0.01 | 0.01 |
| Tea Tree Oil | 0.01 | 0.01 |
| Shea Butter | 0.01 | 0.01 |
| Preservative (VERSTATIL^{®} TBO) | 1.5 | 1.5 |
| Fragrance | 0.3 | 0.3 |
| Water | 71.17 | 65.17 |
| Sodium polyitaconate | - | 6.0 |

The compositions were made and the human hair wig adhered to the bald-headed mannequin. Adhesion was tested by inverting the mannequin head and applying a weight or physically pulling on the wig to see if the adhesion would release.

No adhesion was observed in the composition containing cellulose and no sodium polyitaconate at ambient or at an elevated temperature of 45°C.

### Example 6

In this example, an experiment was conducted to determine if com syrup and sodium itaconate alone provided an adhesive effect. Two adhesive compositions were made as shown in Table 8 one containing corn syrup and sodium polyitaconate (Sample 28) and one containing both sodium polyitaconate and water (Sample 29). In this example, in vitro testing was conducted using bald headed mannequins and human hair wigs both at an ambient temperature of 25°C and at an elevated temperature of 40°C.

**Table 8**

| **Material** | **Sample 28** | **Sample 29** |
|---|---|---|
| Com Syrup | 20.0 | - |
| Hydroxyethyl Cellulose | 2.0 | 2.0 |
| Glycerine | 5.0 | 5.0 |
| Borage Oil | 0.01 | 0.01 |
| Tea Tree Oil | 0.01 | 0.01 |
| Shea Butter | 0.01 | 0.01 |
| Preservative (VERSTATIL^{®} TBO) | 1.5 | 1.5 |
| Fragrance | 0.3 | 0.3 |
| Water | 65.17 | 85.17 |
| Sodium polyitaconate | 6.0 | 6.0 |

The compositions were made and the human hair wig adhered to the bald-headed mannequin. Adhesion was tested by inverting the mannequin head and applying a weight or physically pulling on the wig to see if the adhesion would release.

No synergy for the composition containing com syrup and sodium polyitaconate was observed at ambient or at an elevated temperature of 45°C.

### Example 7

In this example, an experiment was conducted to determine if sodium polyitaconate alone provided an adhesive effect. Two adhesive compositions were made as shown in Table 9, one containing sodium polyitaconate (Sample 30) and one containing just sodium polyitaconate and water (Reference Sample 31). In this example, in vitro testing for 24-48 hours was conducted using bald headed mannequins and human hair wigs both at an ambient temperature of 25°C and at an elevated temperature of 45°C.

**Table 9**

| **Material** | **Sample 30** | **Reference Sample 31** |
|---|---|---|
| Com Syrup | 20.0 | - |
| Hydroxyethyl Cellulose | 2.0 | - |
| Glycerine | 5.0 | - |
| Borage Oil | 0.01 | - |
| Tea Tree Oil | 0.01 | - |
| Shea Butter | 0.01 | - |
| Preservative (VERSTATIL^{®} TBO) | 1.5 | - |
| Fragrance | 0.3 | - |
| Water | 65.17 | 94.0 |
| Sodium polyitaconate | 6.0 | 6.0 |

The compositions were made and the human hair wig adhered to the bald-headed mannequin. Adhesion was tested by inverting the mannequin head and applying a weight or physically pulling on the wig to see if the adhesion would release.

Increased adhesion was observed with the composition of Sample 23 and water, but synergy was still observed for Reference Sample 24 with the composition containing only sodium polyitaconate and water. It was observed that there were no adverse softening affects from the additional components added into the composition at ambient or at an elevated temperature of 45°C.

Other useful tests include an in-use test where wig wearers wear the wig for 3 days with testing after day 1, a sweat and humidity test where wig wearers sweat for 30 minutes, a color safe test for natural and synthetic hair to ensure the adhesive does not damage or alter hair color, in use test for braided or weaved hair and a frizz test.

Except where otherwise explicitly indicated, all numbers in this description indicating amounts of material or conditions of reaction, physical properties of materials and/or use are to be understood as modified by the word "about." All amounts are by weight of the final composition, unless otherwise specified.

It should be noted that in specifying any range of concentration or amount, any particular upper concentration can be associated with any particular lower concentration or amount as well as any subranges consumed therein. In that regard, it is noted that all ranges disclosed herein are inclusive of the endpoints, and the endpoints are independently combinable with each other (e.g., ranges of "up to 25% by weight, or, more specifically, 5% by weight to 20% by weight, in inclusive of the endpoints and all intermediate values of the ranges of 5% by weight to 25% by weight, etc.). "Combination is inclusive of blends, mixtures, alloys, reaction products, and the like. Furthermore, the terms "first", "second", and the like herein do not denote any order, quantity, or importance, but rather are used to distinguish one element from another. The terms "a" and "an" and "the" herein do not denote a limitation of quantity and are to be construed to cover both the singular and the plural, unless otherwise indicated herein or clearly contradicted by context. The suffix "(s)" as used herein is intended to include both the singular and the plural of the term it modifies, thereby including one or more of the term (e.g., the film(s) includes one or more films). Reference throughout the specification to "one embodiment", "one aspect", "another embodiment", "another aspect", "an embodiment", "an aspect" and so forth means that a particular element (e.g., feature, structure, and/or characteristic) described in connection with the embodiment or aspect is included in at least one embodiment or aspect described herein and may or may not be present in other embodiments or aspects. In addition, it is to be understood that the described elements may be combined in any suitable manner in the various embodiments or aspects.

For the avoidance of doubt the word "comprising" is intended to mean "including" but not necessarily "consisting of" or "composed of." In other words, the listed steps, options, or alternatives need not be exhaustive.

The disclosure of the invention as found herein is to be considered to cover all aspects as found in the claims as being multiply dependent upon each other irrespective of the fact that claims may be found without multiple dependency or redundancy. Unless otherwise specified, numerical ranges expressed in the format "from x to y" are understood to include x and y. In specifying any range of values or amounts, any particular upper value or amount can be associated with any particular lower value or amount. All percentages and ratios contained herein are calculated by weight unless otherwise indicated. The various features of the present invention referred to in individual sections above apply, as appropriate, to other sections *mutatis mutandis.* Consequently, features specified in one section may be combined with features specified in other sections as appropriate. Any section headings are added for convenience only and are not intended to limit the disclosure in any way.

## Claims

1. An adhesive composition for adhering an object such as a wig, extension or weave to human skin, comprising:
about 2.5 to about 12% by weight of sodium polyitaconate;
about 0.5 to about 1.5% by weight of a preservative; and
at least 10% by weight of water.

2. The adhesive composition of Claim 1, containing about 4 to about 10% by weight of sodium polyitaconate, preferably containing about 5 to about 7% by weight of sodium polyitaconate.

3. The adhesive composition of any of the preceding claims, wherein the preservative comprises benzoic acid, citric acid, salicylic acid, phenoxyethanol, dehydroacetic acid and salts thereof, chlorphenesin, diols, preferably caprylyl glyclol, diol/glycol compounds, caprylhydroxamic acid, or a combination thereof.

4. The adhesive composition of any of the preceding claims, further comprising a thickening agent.

5. The adhesive composition of Claim 4, wherein the thickening agent comprises cellulose, polysaccharide gum, or a combination thereof, preferably wherein the thickening agent comprises hydroxy cellulose, hydroxyethyl cellulose, hydroxyethyl methylcellulose, methyl cellulose, xanthan gum, guar gum, or a combination thereof.

6. The adhesive composition of any of the preceding claims, further comprising a conditioning agent.

7. The adhesive composition of Claim 6, wherein the conditioning agent comprises glycerine, com syrup, glycols, urea or a combination thereof, preferably wherein the conditioning agent comprises butylene glycol, propanediol, or a combination thereof.

8. The adhesive composition of any of the preceding claims, wherein the adhesive composition has a viscosity of 60,000 to 90,000 centipoise.

9. The adhesive composition of any of the preceding claims, wherein a pH of the adhesive composition is 5.5.

10. Use of the adhesive composition of any of the preceding claims in a lotion, butter, powder, gel, liquid, spray, aerosol, non-aerosol, or a combination thereof.

11. A method of adhering an object to human skin, comprising:
applying the adhesive composition of any of Claims 1 to 9 to human skin; and
allowing the adhesive composition to dry for a period of time, forming an adhesive bond with the human skin.

12. The method of Claim 11, wherein the object comprises an object to be adhered to a human scalp, preferably wherein the object comprises a wig, a weave, a hair extension, or a combination thereof.

13. The method of Claim 11 or Claim 12, wherein adhesive bond lasts for at least one week.

## Patentansprüche

1. Klebstoffzusammensetzung zum Ankleben eines Objekts, wie einer Perücke, einer Extension oder eines Weaves, an menschliche Haut, umfassend:
etwa 2,5 bis etwa 12 Gewichts-% Natriumpolyitaconat;
etwa 0,5 bis etwa 1,5 Gewichts-% eines Konservierungsmittels; und
mindestens 10 Gewichts-% Wasser.

2. Klebstoffzusammensetzung nach Anspruch 1, enthaltend etwa 4 bis etwa 10 Gewichts-% Natriumpolyitaconat, bevorzugt etwa 5 bis etwa 7 Gewichts-% Natriumpolyitaconat.

3. Klebstoffzusammensetzung nach einem der vorhergehenden Ansprüche, wobei das Konservierungsmittel Benzoesäure, Zitronensäure, Salicylsäure, Phenoxyethanol, Dehydroessigsäure und Salze davon, Chlorphenesin, Diole, vorzugsweise Caprylyl-Glycol, Diol/Glycol-Verbindungen, Caprylhydroxamsäure oder eine Kombination davon umfasst.

4. Klebstoffzusammensetzung nach einem der vorhergehenden Ansprüche, die außerdem ein Verdickungsmittel umfasst.

5. Klebstoffzusammensetzung nach Anspruch 4, wobei das Verdickungsmittel Cellulose, Polysaccharidgummi oder eine Kombination davon umfasst, wobei das Verdickungsmittel vorzugsweise Hydroxycellulose, Hydroxyethylcellulose, Hydroxyethylmethylcellulose, Methylcellulose, Xanthangummi, Guargummi oder eine Kombination davon umfasst.

6. Klebstoffzusammensetzung nach einem der vorhergehenden Ansprüche, die außerdem ein Konditionierungsmittel umfasst.

7. Klebstoffzusammensetzung nach Anspruch 6, wobei das Konditionierungsmittel Glycerin, Maissirup, Glycole, Harnstoff oder eine Kombination davon umfasst, wobei das Konditionierungsmittel bevorzugt Butylenglycol, Propandiol oder eine Kombination davon umfasst.

8. Klebstoffzusammensetzung nach einem der vorhergehenden Ansprüche, wobei die Klebstoffzusammensetzung eine Viskosität von 60.000 bis 90.000 Centipoise aufweist.

9. Klebstoffzusammensetzung nach einem der vorhergehenden Ansprüche, wobei der pH-Wert der Klebstoffzusammensetzung 5,5 beträgt.

10. Verwendung der Klebstoffzusammensetzung nach einem der vorhergehenden Ansprüche in einer Lotion, Butter, einem Puder, einem Gel, einer Flüssigkeit, einem Spray, einem Aerosol, einem Nicht-Aerosol oder einer Kombination davon.

11. Verfahren zum Ankleben eines Gegenstandes an menschliche Haut, umfassend:
Auftragen der Klebstoffzusammensetzung nach einem der Ansprüche 1 bis 9 an menschliche Haut und
Trocknenlassen der Klebstoffzusammensetzung für eine Zeit lang, wodurch eine Klebeverbindung mit der menschlichen Haut entsteht.

12. Verfahren nach Anspruch 11, wobei der Gegenstand einen Gegenstand umfasst, der an eine menschliche Kopfhaut geklebt wird, wobei der Gegenstand bevorzugt eine Perücke, ein Weave, eine Haarextension oder eine Kombination davon umfasst.

13. Verfahren nach Anspruch 11 oder Anspruch 12, wobei die Klebeverbindung mindestens eine Woche lang hält.

## Revendications

1. Composition adhésive pour faire adhérer un objet tel qu'une perruque, une extension ou un tissage à de la peau humaine, comprenant :
environ 2,5 à environ 12 % en poids de poly(itaconate de sodium) ;
environ 0,5 à environ 1,5 % en poids d'un conservateur ; et
au moins 10 % en poids d'eau.

2. Composition adhésive selon la revendication 1, contenant environ 4 à environ 10 % en poids de poly(itaconate de sodium), de préférence contenant environ 5 à environ 7 % en poids de poly(itaconate de sodium).

3. Composition adhésive selon l'une quelconque des revendications précédentes, dans laquelle le conservateur comprend de l'acide benzoïque, de l'acide citrique, de l'acide salicylique, du phénoxyéthanol, de l'acide déshydroacétique et ses sels, de la chlorphénésine, des diols, de préférence du caprylylglycol, des composés de diol/glycol, de l'acide caprylhydroxamique, ou une combinaison de ceux-ci.

4. Composition adhésive selon l'une quelconque des revendications précédentes, comprenant en outre un agent épaississant.

5. Composition adhésive selon la revendication 4, dans laquelle l'agent épaississant comprend de la cellulose, de la gomme de polysaccharide, ou une combinaison de celles-ci, de préférence dans laquelle l'agent épaississant comprend de l'hydroxycellulose, de l'hydroxyéthylcellulose, de l'hydroxyéthylméthylcellulose, de la méthylcellulose, de la gomme xanthane, de la gomme de guar, ou une combinaison de celles-ci.

6. Composition adhésive selon l'une quelconque des revendications précédentes, comprenant en outre un agent de conditionnement.

7. Composition adhésive selon la revendication 6, dans laquelle l'agent de conditionnement comprend de la glycérine, du sirop de maïs, des glycols, de l'urée ou une combinaison de ceux-ci, de préférence dans laquelle l'agent de conditionnement comprend du butylèneglycol, du propanediol, ou une combinaison de ceux-ci.

8. Composition adhésive selon l'une quelconque des revendications précédentes, laquelle composition adhésive a une viscosité de 60 000 à 90 000 centipoises.

9. Composition adhésive selon l'une quelconque des revendications précédentes, dans laquelle le pH de la composition adhésive est de 5,5.

10. Utilisation de la composition adhésive de l'une quelconque des revendications précédentes dans une lotion, un beurre, une poudre, un gel, un liquide, un spray, un aérosol, un non-aérosol, ou une combinaison de ceux-ci.

11. Procédé pour faire adhérer un objet à de la peau humaine, comprenant :
l'application de la composition adhésive de l'une quelconque des revendications 1 à 9 à de la peau humaine ; et
le fait de laisser la composition adhésive sécher pendant un certain temps, en formant une liaison adhésive avec la peau humaine.

12. Procédé selon la revendication 11, dans lequel l'objet comprend un objet devant adhérer au cuir chevelu humain, de préférence dans lequel l'objet comprend une perruque, un tissage, une extension capillaire, ou une combinaison de ceux-ci.

13. Procédé selon la revendication 11 ou la revendication 12, dans lequel la liaison adhésive dure au moins une semaine.
